# EUROPEAN PATENT SPECIFICATION

(11) **EP 2 122 021 B1**
(45) Date of publication and mention of the grant of the patent: **08.01.2014**
(21) Application number: 08730690.8
(22) Date of filing: 26.02.2008
(51) Int. Cl.: D01F 8/14, A61B 17/06, A61L 17/12

(54) **CO-EXTRUDED TISSUE GRASPING MONOFILAMENT**
KOEXTRUDIERTES MONOFILAMENT ZUR GEWEBEERGREIFUNG
MONOFILAMENT CO-EXTRUDÉ DE SAISIE DE TISSU

(30) Priority: 09.03.2007 US 684027
(43) Date of publication of application: 25.11.2009
(73) Proprietor: Ethicon, Inc, Somerville, NJ 08876-0151 (US)
(72) Inventor: CHEN, Gaoyuan, Hillsborough, NJ 08844 (US); YUAN, J. Jenny, Branchburg, New Jersey 08853 (US); MATRUNICH, James A., Mountainside, New Jersey 07092 (US)
(74) Representative: Schohe, Stefan
(86) International application number: PCT/US2008/054934
(87) International publication number: WO 2008/112417

(56) References cited:
- WO-A-98/52473
- WO-A-03/017850
- WO-A-03/083191
- US-A- 3 458 390
- US-A- 3 700 544
- US-A1- 2007 005 109
- US-B2- 6 420 027

## Description

### Field of the Invention

The present invention relates generally to the field of surgical medical devices, and more particularly to tissue grasping monofilaments comprising at least two co-extruded distinct materials.

### Background of the Invention:

Many wound and surgical incisions are closed using surgical sutures or some other surgical closure device. With regard to surgical sutures, various types of barbed sutures have been developed and/or discussed in literature in an effort to help prevent slippage of the suture and/or eliminate at least some knot-tying. With such known barbed sutures, the configuration of the barbs, such as barb geometry (barb cut angle, barb cut depth, barb cut length, barb cut distance, etc.) and/or the spatial arrangement of the barbs, will likely affect the tensile strength and/or holding strength of the suture. There is much prior art focusing on these features, mostly in the context of barbs that are cut into the suture shaft or suture core. In most known monofilament cut, barbed sutures, the tensile strength of a barbed suture is significantly less than a non-barbed suture of equivalent size. This is due to the fact that escarpment of barbs into a monofilament, depending on the barb cut depth, reduces the straight pull tensile strength since the effective suture diameter is decreased. Further, unlike conventional sutures that disproportionately place tension directly at the knots, barbed sutures tend to spread out the tension more evenly along the suture length, including at the location of the barbs. It is therefore critical for the monofilament, at the location of the barbs, to have sufficient tensile strength, and also critical for the barbs themselves to be sufficiently strong to resist breakage or peeling.
Most monofilament barbed sutures are made of relatively soft polymeric materials, thus providing a limit on the stiffness of the barbs. For any given suture size, it is difficult to form barbs large enough and strong enough to catch tissues without bending, slippage or breakage, and without adversely affecting the strength of the suture. The holding strength and tensile strength can be increased by use of a stiffer material for the suture, but any increase in stiffness leads to a decrease in the flexibility of the suture, which is undesirable.

International Application WO03/083191 A1 discloses a monofilament suture with a round cross-section. The suture comprises a first polymer with a high Young's modulus and a second polymer with a low Young's modulus. The suture is formed so that the first polymer surrounds the second polymer.

International application WO98/52473 discloses a method for producing one-way sutures. Here, cutting plates are used to form barbs in the suture with the raw suture being nylon.

US 3,458,390 A discloses a method for producing a filament for textiles, knitted fabrics and for piles of carpets. The filament comprises two constituents. In a cross-sectional view, one of the constituents forms a protruding pattern into the other constituent.

For the foregoing reasons, there is a need for a tissue grasping monofilament having an improved combination of strength and flexibility.

### SUMMARY OF INVENTION

The invention solves this problem by providing a monofilament according to claim 1 and a method according to claim 12. In particular, the present invention provides a co-extruded, tissue grasping monofilament having a core made of a first material and extending along a length of the monofilament, and a plurality of tissue grasping elements extending outwardly from the core at least along a predetermined portion of the length of the monofilament. The plurality of tissue grasping elements are made of a second, different material having a greater stiffness than the first material. In one aspect of the invention, the monofilament may be of a size suitable for use as a surgical suture.
According to one embodiment, the second material substantially surrounds the core. In yet another embodiment, the plurality of tissue grasping elements each have a base portion and a distal end portion, with the base portion being embedded within the core. The base portion may further include one or more projections extending laterally outwardly therefrom that assist in mechanically coupling the tissue grasping elements with the core. Further, the cross-section of the plurality of tissue grasping elements may decreases from the proximal end to the distal tip located farthest from the core.

The core may have a substantially uniform cross-section along the length of the monofilament, and may further have a shape that is circular, oval, triangular or polygonal.

In further alternative embodiments, the first material may have an initial modulus of less than or equal to about 2.76 GPa (400 kpsi), and/or the second material may have an initial modulus of at least about 3.45 GPa (500 kpsi).

Further, the first material may be a polymeric material such as polyethylene terephthalate, or polymers or copolymers of lactide and glycolide, which may further be 95/5 copolymer of poly(lactide-co-glycolide) or 90/10 copolymer of poly(glycolide-co-lactide). The second material may be a polymeric material such as polypropylene, polydioxanone, or copolymers of poly(glycolide-co-caprolactone), which may further be a 75/25 blocked copolymer of poly(glycolide-co-caprolactone).

According to yet another embodiment the monofilament is formed by coextrusion of the first and second materials.

Also provided is a method for forming a tissue grasping monofilament including the steps providing a first material having a first stiffness in its solid state, providing a second material having a second, different stiffness in its solid state that is greater than that of the first material, melting the first material and extruding the melted first material through a first die having a predetermined shape to form a first melt stream having substantially the predetermined shape, melting the second material and introducing the melted second material into a merging chamber having the first melt stream passing therethrough such that the second material substantially surrounds said first melt stream, extruding the first melt stream surrounded by the melted second material together through a second die having a predetermined shape with an outer periphery greater than an outer periphery of the first die and with at least one ridge extending outwardly beyond the outer periphery of the first die, and cooling said first and second materials to form a solid monofilament. The method may further include the step(s) of drawing the cooled monofilament to form an oriented monofilament, and/or, following cooling, forming tissue grasping elements along a predetermined length of the second material by removing material from the at least one ridge formed of the second material.

In one embodiment, the predetermined shape of the first die is substantially oval or circular.

In yet another embodiment, the first material has an initial modulus of less than or equal to about 2.76 GPa (400 kpsi) and the second material has an initial stiffness of at least about 3.45 GPa (500 kpsi).

The first material may further be a polymeric material such as polyethylene terephthalate or polymers or copolymers of lactide and glycolide, and the second material may further be a polymeric material such as polypropylene, polydioxanone, or copolymers of poly(glycolide-co-caprolactone).

A further method is provided including the steps of providing a first material having a first stiffness in its solid state, providing a second different material having a second stiffness in its solid state that is greater than that of the first material, melting the first and second materials, and co-extruding the first and second materials to form a monofilament wherein the first material forms a core of the monofilament and the second material forms one or more ridges extending outwardly beyond an outer periphery of the core.

According to this method the second material of the co-extruded monofilament may further substantially surround the core.

In yet another embodiment, a base portion of each of the plurality of ridges may further be embedded within the core and a distal end portion of each of the plurality of ridges extend outwardly beyond the outer periphery of the core. The base portion each of the plurality of ridges may further include one or more projections extending laterally outwardly therefrom.

In yet another embodiment, the method further includes forming a plurality of tissue grasping elements in the one or more ridges by removing material therefrom at predetermined locations.

In additional alternative embodiments, the core of the monofilament may have a substantially oval or circular shape, and/or the first material may have an initial modulus of less than or equal to about 2.76 GPa (400 kpsi), and the second material may have an initial stiffness of at least about 3.45 GPa (500 kpsi).

The first material may further be a polymeric material such as polyethylene terephthalate or polymers or copolymers of lactide and glycolide, and the second material may be a polymeric material such as polypropylene, polydioxanone or copolymers or poly(glycolide-co-caprolactone).

### Brief Description of the Drawings

The invention will now be described in more detail with reference to the accompanying drawings, in which:
Figure 1a is a schematic illustration of an exemplary co-extrusion process that can be used to form monofilaments according to the present invention;
Figure 1b is a cross-section of one embodiment of a monofilament of the present invention;
Figures 1c and 1d are perspective views of the monofilament of Figure 1b before and after tissue grasping elements are formed;
Figures 1e-1f are cross-sectional views illustrating alternate embodiments of the monofilament of the present invention;
Figure 2 is a schematic illustration of an exemplary drawing process that can be used to form monofilaments according to the present invention;
Figure 3a-3d are cross-sectional views of various embodiments of a monofilament according to the present invention wherein the tissue grasping elements are at least partially embedded within the core;
Figure 4 illustrates a cross-section of an embodiment of a monofilament according to the present invention wherein the tissue grasping elements are formed on and adhere to the outer periphery of the core; and
Figure 5 illustrates an exemplary cut that can be used in forming tissue grasping elements on a monofilament according to the present invention.

### Detailed Description:

By way of background and as those skilled in the art recognize, "extrusion" typically refers to a polymer processing technique in which a polymer is melted and pressurized in an extruder, and fed through a die in a continuous stream. For purposes of the present application, the term "co-extrusion" refers to a process where two or more different materials, such as polymers, are melted in separate extruders with both melt streams fed through a co-extrusion die wherein they are joined to form a single molten strand. Further, the term "stiffness" as used herein refers the load required to deform a material, which is measured by the slope of the stress-strain curve. The initial slope of the stress-strain curve (typically from 0.5% - 1.5% strain range) is also termed as Young's Modulus or the initial modulus, which is the measure of stiffness used herein.

Tissue grasping monofilament medical devices according to the present invention comprise at least two different components that are co-extruded. The term "different" as used herein is intended to cover both distinctly different materials having fundamentally different chemical formulas and structures, or materials having similar chemical formulas and structures, but different molecular weights and thus potentially different physical properties. The first component forms a core or shaft and the second component forms the tissue grasping elements, or one or more " ridges" extending substantially lengthwise along a predetermined length of the filament, and out of which the tissue grasping elements are formed by cutting or otherwise removing portions of the ridge. The cross-section of the core may be any shape including, but not limited to, round, oval, triangle, square or rectangular. The cross-section of the ridge and ultimately the tissue grasping elements can also be of substantially any shape suitable to increase the holding strength of the monofilament. Particularly suitable configurations of the ridge are triangular or various other shapes that have a wider base than distal end. The core and the ridges may be coupled simply by adherence of the two dissimilar materials together during the co-extrusion process, or may be physically reinforced by complementary interlocking shapes as will be described further below. By co-extruding two different materials and optimally selecting the materials as described herein, a tissue grasping monofilament can be achieved having both improved strength of the tissue grasping elements, and an improved combination of tensile strength and flexibility.

The two materials may be made from various suitable biocompatible materials, such as absorbable or non-absorbable polymers. The two materials may have different properties, such as modulus, strength, in vivo degradation rates, so that the desired properties for overall performance of the tissue grasping monofilament device and the capability of the tissue grasping elements to engage and maintain wound edges together can be tailored. Preferably, the first component is a relatively soft material having an initial modulus of no greater than about 2.76GPa (400kpsi), and the second component is a stiffer material having an initial modulus of at least about 3.45GPa (500kpsi). Preferable materials for the second component include, but are not limited to, polyethylene terephthalate, polymers or copolymers of lactide and glycolide, and more preferably 95/5 copolymer of poly (lactide-co-glycolide), 90/10 copolymer of poly (glycolide-co-lactide), and materials for the first component include, but are not limited to, polypropylene, polydioxanone, copolymers of poly (glycolide-co-caprolactone).

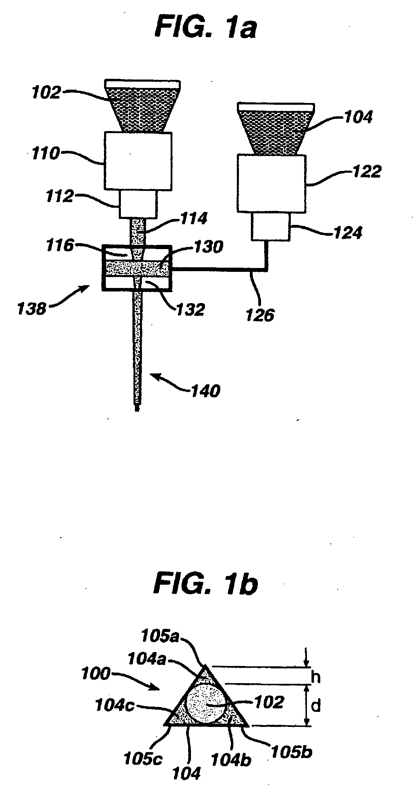

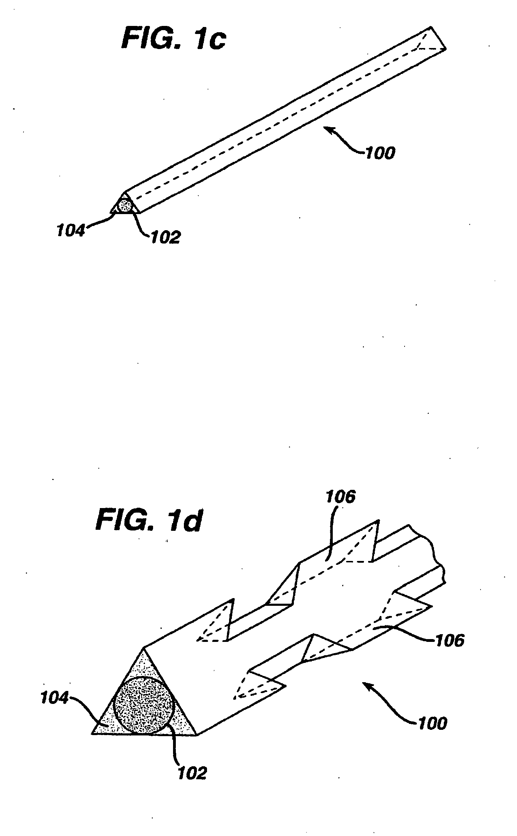

Figures 1b-d illustrate one exemplary embodiment of a co-extruded tissue grasping monofilament 100 according to the present invention. In this embodiment, the first component 102 is made of polydioxanone (PDS), and the second component 104 is made of polylactide (PLA) and polyglycolide (PGA) or 95/5 PLA/PGA copolymers (a stiffer material with a higher initial modulus). The second component has a substantially triangular overall outer perimeter forming first, second and third 104a, 104b, 104c ridges extending outwardly from the core 102. Tissue grasping elements 106 subsequently cut into the ridges are shown in Fig. 1d. With a co-extruded monofilament wherein the second material has a greater stiffness, the holding strength of the tissue grasping elements is greater due to the greater stiffness. In the illustrated embodiment, the core is substantially circular in cross-section and has an outer diameter d of approximately 0.05-0.76 mm (2-30 mil), preferably 0.127-0.635 mm (5-25 mil). Further, each ridge and resulting tissue grasping elements projects outwardly from the core to a distal tip 105a, 105b, 105c a distance h of approximately 0.0762-1.27 mm (3.50 mil), preferably 0.2032-0.889 mm (8-35 mil).

Referring now to Fig. 1a, one exemplary process for making a co-extruded monofilament of the type shown in Figs. 1b-d will now be described in detail. The first component, which as indicated can be PDS, is melted in a first extruder 110, metered and pressurized through a gear pump 112. The pressurized polymer melt stream 114 (which is inside a heated metal block or a transfer tube, not shown) passes through an upper die 116 of a shape suitable to form the desired cross-section of the core, in this case circular. The second component (i.e., PLA) 104 is melted in a second extruder 122, metered, and pressurized through the gear pump 124. The second pressurized polymer melt stream 126 (inside a heated transfer tube, not shown) enters a merging chamber 130 in the co-extrusion die block 138 between the upper die 116 and a lower die 132. More specifically, as used herein the term "merging chamber" refers to the portion of the co-extrusion die block 138 where the melt streams of the first and second components merge before being extruded together through the bottom or lower die 132. At a given temperature, the lower modulus material has a lower viscosity, which aids in its ability to flow around the core component before entering the lower die. The merged stream 134 of the two components passes through the lower die 132 of a predetermined shape (in this case triangular) to form the desired overall cross-section of the co-extruded monofilament 140.

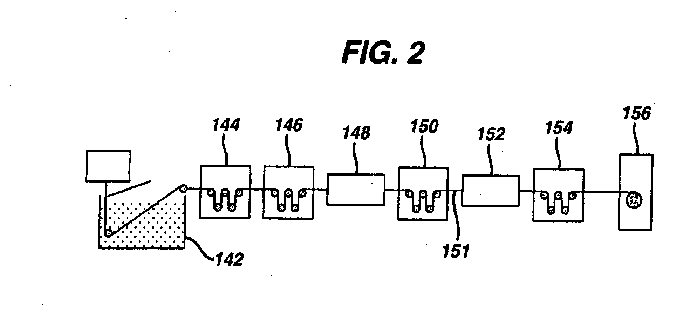

The co-extruded molten monofilament strand 140 exiting the co-extrusion die block 138 is quenched and solidified in a liquid bath 142 as illustrated in Fig. 2, to quickly preserve the shape of the extrudate. The solidified dual-component monofilament strand is then passed through a first set of godet rolls 144 at a constant speed and then drawn or stretched preferably to 2-10 times its original length with the second set of feeding or godet rolls 146 running at a faster speed. As is well known, drawing or stretching (as opposed to injection molding techniques) improves strength by orienting molecules along the axis of the fiber. The drawn strand may be drawn for the second time with the third set of rolls 150 to reach the maximum stable draw ratio to optimize the tensile properties. During the drawing process, the monofilament can be heated with one or several of the feeding rolls and/or through a hot oven 148. The fully drawn monofilament 151 may then be relaxed by passing through a heated relaxation oven 152 and onto another set of rolls 154 running at a slightly slower speed before taking up with winding device 156.

The co-extrusion process described above, in combination with natural adherence between the two materials, mechanically couples the two components to result in a suitable co-extruded monofilament. The core of the first, less stiff material allows for good overall flexibility of the monofilament, while the second, stiffer material into which the tissue grasping elements are formed allows for stronger tissue grasping elements leading to better holding strength for the monofilament. Finally, because the suture core 102 remains intact, tensile strength is not adversely affected.

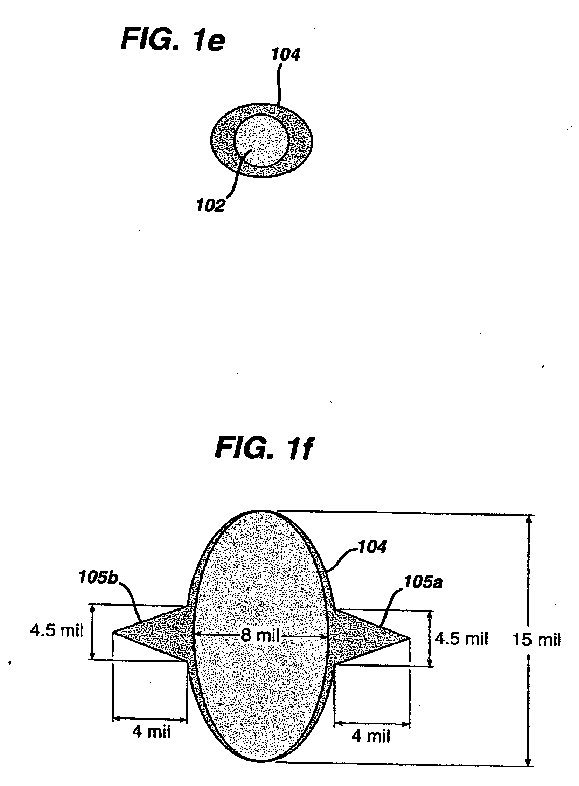

Although a substantially triangular overall cross-section is illustrated in Fig. 1b, it is to be understood that any suitable cross-section can be used and achieved with coextrusion, such as, but not limited to, circular or oval as shown in Figs 1e and 1f, or any suitable polygonal cross-section. The cross-section of the core may be varied as well.

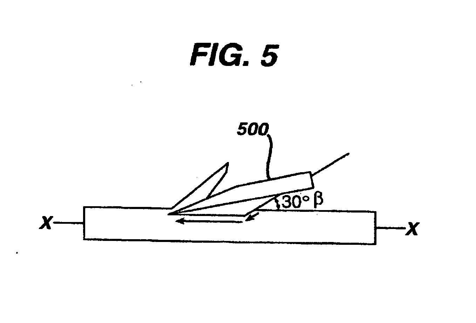

As previously indicated, the tissue grasping elements can be formed in the ridges in any suitable configuration and by any suitable manner known to those skilled in the art, such as cutting by knife, laser or other device, stamping, punching, press forming or the like. For example, in one embodiment the tissue grasping elements are formed by cutting with a suitable cutting blade or knife. The desired number of acute, angular cuts are made directly into the ridges of the co-extruded monofilament. Figure 5 illustrates an exemplary cut, where the cutting blade 500 first cuts into the ridge at an angle β of approximately 30 degrees relative to the longitudinal axis x-x of the monofilament, to a depth approximately equal to or preferably less than the height of the ridges, and subsequently further cuts into the monofilament for a distance of approximately 50% ∼100% of the height of the ridges at an angle of approximately 0 degrees. To facilitate this cutting, the monofilament is typically placed and held on a cutting vice or the like. A template may also be used to help guide the cutting blade. As the ridges protrude from the core, an alternate means for cutting the tissue grasping elements is to slice across the ridges from one side to the other, thus making it a one motion movement cutting and increasing efficiency. The blade will take the shape of the tissue grasping element configuration with the cutting blade on the side instead of in the front. Also, since the tissue grasping element configuration is pre-determined by the shape of the blades, the changes can easily be made to the machine if changes are desired. As indicated, material can be removed from the ridges by other suitable means such as laser cutting or stamping.

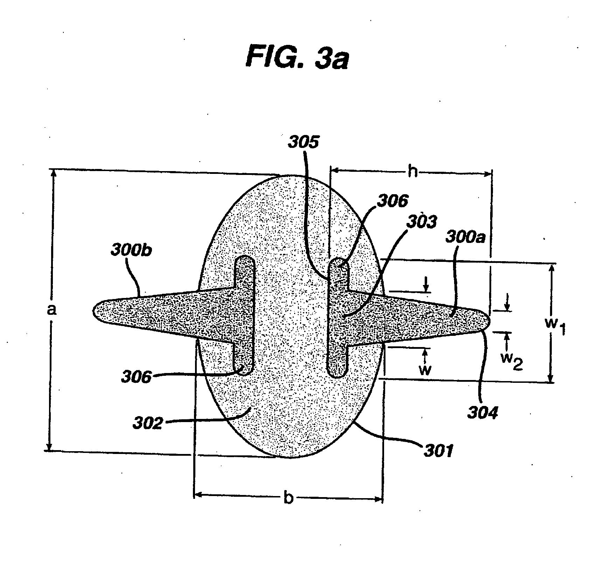

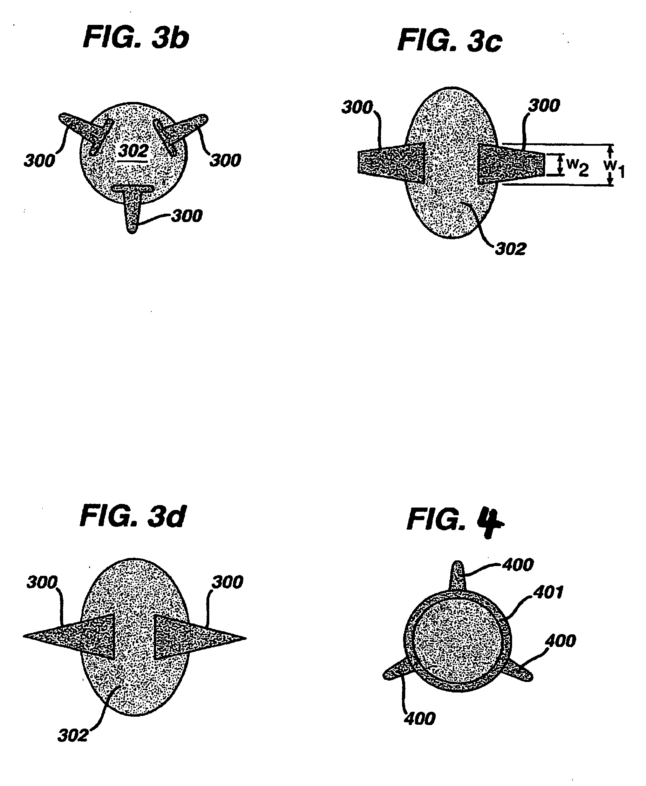

Referring now to Figs. 3a-d, in alternate embodiments according to the present invention, the second component from which the tissue grasping elements are formed does not surround the core, but rather is mechanically coupled with the core and projects outwardly therefrom. For example, as shown in Fig. 3a, first and second ridges 300a, 300b (within which the tissue grasping elements are subsequently formed) extend outwardly from the core 302, but a base portion 303 at a proximal end thereof is embedded within the core. Preferably, each is configured to provide additional mechanical resistance against pulling the tissue grasping elements out of the core. In Figs. 3a-c, the ridges include the base portion 303 that is larger in width w₁ and cross-section than the width w₂ and cross-section of the distal tip portion 304. The base portion may include additional extensions or projections 306 that extend laterally outward and assist in mechanically locking the projection to the core. As stated, embedding the ridges into the core provides additional security through "mechanical locking" between the ridge material and core material. The two ridges preferably are placed along the short axis of the oval core if the core is oblong, so as to minimally affect overall stiffness of the monofilament.
Further, in the illustrated exemplary embodiment, the overall dimension of the core is approximately 0.1016-1.016 mm (4-40 mil), preferably 0.381 mm (15 mil) (dimension a) by approximately 0.0508-0.508 mm (2-20 mil), preferably 0.203 mm (8 mil) (dimension b), and dimensions w, w₁ and w₂ are approximately 0.0254-0.254 mm (1-10 mil), preferably 0.1016 mm (4 mil), 0.0508-0.508 mm (2-20 mil), and preferably 0.2032 mm (8 mil), and 0.01016-0.1016 mm (0.4-4 mil), preferably 0.0381 mm (1.5 mil).

As further shown in Figures 3b-3d, the number of ridges 300 and/or their configurations can vary to best suit the desired product features in a given surgical application. In addition, the core can take circular and non-circular cross-sections to accommodate the number of ridges, mechanical properties of the filaments, and the extrusion process. Further, similar type ridges 400 extending from the outer periphery of the core can be connected by a relatively thin membrane or covering 401 of the same material that surrounds or substantially surrounds the core as shown in Fig. 4.

The following are detailed representative examples of co-extruded, tissue grasping monofilaments of the present invention which are exemplary only, as the present invention is not intended to be limited other than by the appended claims.

### Example 1

A nonabsorbable tissue grasping monofilament substantially of the configuration shown in Fig. 1b was formed using the coextrusion process shown and described above in connections with Figures 1a and 2. Polypropylene (PP) was used as the first component with has an initial modulus of 1.627GPa (236 kpsi) in the oriented fiber of the homopolymer. Polyethylene terephthalate (PET) with an initial modulus of 14.093GPa (2044 kpsi), was used for the second component.

As shown in Fig. 1a, the first component, PP, was melted in a first extruder 110, where the extruder barrel had three temperature zones maintained, respectively, at 180, 195 and 210 °C. The melted polymer stream was metered and pressurized through a gear pump 112 and the pressurized polymer melt stream 114 passed through a circular upper die 116 to form a circular core. The second component (PET) was melted in a second extruder 122 maintained at a constant temperature of 285 °C in all three zones. The melt flow was then metered, and pressurized through the gear pump 124. The second pressurized polymer PET melt stream 126 entered a merging chamber 130 in the co-extrusion die block 138 between the upper die 116 and a lower die. The merged stream 134 of the two components passes through the lower die 132 of a triangular shape to form a triangular overall cross-section of the co-extruded monofilament 140.

The co-extruded molten PP/PET monofilament strand 140 exiting the coextrusion die block 138 was quenched and solidified in a liquid bath 142 as illustrated in Fig. 2. The solidified PP/PET dual-component monofilament strand was then passed through a first set of godet rolls 144, the last two of which were heated at a temperature of 122 °C. The feeding speed was 0.62 m/s (122 feet per minute (fpm)). The co-extruded monofilament was passed to and drawn with the second set of godet rolls 146 running at a speed of 0.26m/s (50.5 fpm)(no heating was applied). The partially stretched strand was drawn again with the third set of rolls 150 running at 0.29m/s (57 fpm). The total draw ratio was 6.0. The hot oven 148 was 1.83m (six feet) long and was heated at 135 °C. The fully drawn monofilament 151 was relaxed by passing through a 1.83m (six-foot) oven 152 maintained at 135 °C and onto another set of rolls 154 running at a speed of 0.29m/s (57 fpm) before taking up with winding device 156.

Tissue grasping elements were subsequently formed by cutting along the three ridges of essentially PET to form a tissue grasping monofilament having a less stiff, more pliable core while having stiffer, more rigid tissue grasping elements.

### Example 2

A substantially identical configuration and process as Example 1, with the exception that the second component was a 90/10 PGA/PLA random copolymer with an initial modulus of 914 kpsi and an absorption time of 50-70 days. The first component was a 75/25 PGA/PCL block copolymer with an initial modulus of 0.73GPa (106 kpsi) and an absorption time of 91 - 119 days. The two polymer components were found to have been adequately connected via adhesion at their interfaces. Tissue grasping elements were formed as described above.

Although illustrative embodiments of the present invention have been described herein with reference to the accompanying drawings, it is to be understood that the invention is not limited to those precise embodiments and that various other changes and modifications may be effected herein by one skilled in the art without departing from the scope of the claims.

## Claims

1. A co-extruded, tissue grasping monofilament (100), comprising:
a core (102, 302) comprised of a first material and extending along a length of said monofilament; and **characterized by**
a plurality of tissue grasping elements (106, 300) extending outwardly from said core (102, 302) at least along a predetermined portion of the length of the monofilament (100), the plurality of tissue grasping elements (106, 300) being comprised of a second, different material having a greater stiffness than the first material, and in that the first material has an initial modulus of less than or equal to about 2.76 GPa (400 kpsi) and
the second material has an initial modulus of at least about 3.45 GPa (500 kpsi).

2. The monofilament according to claim 1, wherein the monofilament is of a size suitable for use as a surgical suture.

3. The monofilament according to claim 1, wherein the second material substantially surrounds the core (102).

4. The monofilament according to claim 1, wherein the plurality of tissue grasping elements each have a base portion (303) and a distal end portion (304), and wherein the base portion (303) is embedded within the core.

5. The monofilament according to claim 4, wherein the base portion (303) has one or more projections (306) extending laterally outwardly therefrom that assist in mechanically coupling the tissue grasping elements with the core.

6. The monofilament according to claim 1, wherein a cross-section of the plurality of tissue grasping elements (106, 300) decreases from a proximal end thereof to a distal tip thereof located farthest from said core (102, 302).

7. The monofilament according to claim 1, wherein the cross-section of the core (102, 302) is a shape selected from the group consisting of circular, oval, triangular and polygonal.

8. The monofilament according to claim 1, wherein the first material is a polymeric material selected from the group consisting of polyethylene terephthalate, and polymers or copolymers of lactide and glycolide.

9. The monofilament according to claim 8, wherein the copolymers of lactide and glycolide is a polymeric material selected from the group consisting of 95/5 copolymer of poly(lactide-co-glycolide) and 90/10 copolymer of poly(glycolide-co-lactide).

10. The monofilament according to claim 8, wherein the second material is a polymeric material selected from the group consisting of polypropylene, polydioxanone, and copolymers of poly(glycolide-co-caprolactone).

11. The monofilament according to claim 10, wherein the second material is a 75/25 blocked copolymer of poly(glycolide-co-caprolactone).

12. A method for forming a monofilament comprising the steps of:
providing a first material having a first stiffness in its solid state;
providing a second different material having a second stiffness in its solid state that is greater than that of the first material;
melting the first and second materials;
co-extruding the first and second materials to form a monofilament wherein the first material forms a core (102, 302) of the monofilament and **characterized in that** the second material forms one or more ridges (106, 300) extending outwardly beyond an outer periphery of the core, and
wherein the first material has an initial modulus of less than or equal to about 2.76 GPa (400 kpsi), and the second material has an initial stiffness of at least about 3.45 GPa (500 kpsi).

13. The method according to claim 12, wherein the second material of the co-extruded monofilament substantially surrounds the core.

14. The method according to claim 12, wherein a base portion (303) of each of the plurality of ridges (300) is embedded within the core and a distal end portion (304) of each of the plurality of ridges (300) extends outwardly beyond the outer periphery of the core.

15. The method according to claim 14, wherein the base portion (303) each of the plurality of ridges (300) further includes one or more projections (306) extending laterally outwardly therefrom.

16. The method according to claim 12, further comprising forming a plurality of tissue grasping elements in the one or more ridges (300, 400) by removing material therefrom at predetermined locations.

17. The method according to claim 12, wherein the first material is a polymeric material selected from the group consisting of polyethylene terephthalate and polymers or copolymers of lactide and glycolide, and the second material is a polymeric material selected from the group consisting of polypropylene, polydioxanone and copolymers or poly(glycolide-co-caprolactone).

## Patentansprüche

1. Koextrudiertes, Gewebe greifendes Monofil (100), welches Folgendes umfasst:
einen Kern (102, 302), der ein erstes Material umfasst und sich entlang einer Länge des Monofils erstreckt, und **gekennzeichnet durch**
eine Mehrzahl von Gewebe greifenden Elementen (106, 300), die sich von dem Kern (102, 302) zumindest entlang eines vorbestimmten Abschnitts der Länge des Monofils (100) nach außen erstrecken, wobei die Mehrzahl von Gewebe greifenden Elementen (106, 300) ein zweites, anderes Material mit einer größeren Steifigkeit als das erste Material umfassen, und **dadurch**, dass
das erste Material ein Initialmodul von weniger als oder gleich etwa 2,76 GPa (400 kpsi) aufweist und
das zweite Material ein Initialmodul von zumindest etwa 3,45 GPa (500 kpsi) aufweist.

2. Monofil gemäß Anspruch 1, bei dem das Monofil von einer Größe ist, die für eine Verwendung als chirurgisches Nahtmaterial geeignet ist.

3. Monofil gemäß Anspruch 1, bei dem das zweite Material den Kern (102) im Wesentlichen umgibt.

4. Monofil gemäß Anspruch 1, bei dem die Mehrzahl von Gewebe greifenden Elementen jeweils einen Basisabschnitt (303) und einen distalen Endabschnitt (304) aufweisen, und wobei der Basisabschnitt (303) innerhalb des Kerns eingebettet ist.

5. Monofil gemäß Anspruch 4, bei dem der Basisabschnitt (303) einen oder mehrere Vorsprünge (306) aufweist, die sich von dort lateral nach außen erstrecken, und die beim mechanischen Einkoppeln der Gewebe greifenden Elemente mit dem Kern unterstützen.

6. Monofil gemäß Anspruch 1, bei dem ein Querschnitt der Mehrzahl von Gewebe greifenden Elementen (106, 300) von einem proximalen Ende davon zu einer distalen Spitze davon, die sich am weitesten von dem Kern (102, 302) entfernt befindet, abnimmt.

7. Monofil gemäß Anspruch 1, bei dem der Querschnitt des Kerns (102, 302) eine Form ist, die ausgewählt ist aus der Gruppe, die aus rund, oval, dreieckig und polygonal besteht.

8. Monofil gemäß Anspruch 1, bei dem das erste Material ein Polymermaterial ist, das ausgewählt ist aus der Gruppe, die aus Polyethylentherephthalat und Polymeren oder Copolymeren von Lactid und Glycolid besteht.

9. Monofil gemäß Anspruch 8, bei dem die Copolymere von Lactid und Glycolid ein Polymermaterial sind, das ausgewählt ist aus der Gruppe, welche aus 95/5-Copolymer von Poly(lactid-co-glycolid) und 90/10-Copolymer von Poly(glykolid-co-lactid) besteht.

10. Monofil gemäß Anspruch 8, bei dem das zweite Material ein Polymermaterial ist, das ausgewählt ist aus der Gruppe, die aus Polypropylen, Polydioxanon und Copolymeren von Poly(glycolid-co-caprolacton) besteht.

11. Monofil gemäß Anspruch 10, bei dem das zweite Material ein 75/25-Blockcopolymer von Poly(glycolid-co-caprolacton) ist.

12. Verfahren zum Bilden eines Monofils, welches die folgenden Schritte umfasst:
Bereitstellen eines ersten Materials, das in seinem festen Zustand eine erste Steifigkeit aufweist,
Bereitstellen eines zweiten, anderen Materials, das in seinem festen Zustand eine zweite Steifigkeit aufweist, die größer als diejenige des ersten Materials ist,
Schmelzen des ersten und des zweiten Materials,
Koextrudieren des ersten und des zweiten Materials, um ein Monofil zu bilden, wobei das erste Material einen Kern (102, 302) des Monofils bildet, und **dadurch gekennzeichnet, dass** das zweite Material eine oder mehrere Rippen (106, 300), die sich über einen Außenumfang des Kerns hinaus nach außen erstrecken, bildet, und
wobei das erste Material ein Initialmodul von weniger als oder gleich etwa 2,76 GPa (400 kpsi) aufweist und das zweite Material eine Initialsteifigkeit von zumindest etwa 3,45 GPa (500 kpsi) aufweist.

13. Verfahren gemäß Anspruch 12, bei dem das zweite Material des koextrudierten Monofils den Kern im Wesentlichen umgibt.

14. Verfahren gemäß Anspruch 12, bei dem ein Basisabschnitt (303) jeder aus der Mehrzahl von Rippen (300) innerhalb des Kerns eingebettet ist und sich ein distaler Endabschnitt (304) jeder aus der Mehrzahl von Rippen (300) über den Außenumfang des Kerns hinaus nach außen erstreckt.

15. Verfahren gemäß Anspruch 14, bei dem der Basisabschnitt (303) jeder aus der Mehrzahl von Rippen (300) ferner einen oder mehrere Vorsprünge (306), der sich von dort lateral nach außen erstreckt, aufweist.

16. Verfahren gemäß Anspruch 12, welches weiter ein Bilden einer Mehrzahl von Gewebe greifenden Elementen in der einen oder den mehreren Rippen (300, 400) durch ein Entfernen von Material davon an vorbestimmten Orten umfasst.

17. Verfahren gemäß Anspruch 12, bei dem das erste Material ein Polymermaterial ist, das ausgewählt ist aus der Gruppe, die aus Polyethylentherephthalat und Polymeren oder Copolymeren von Lactid und Glycolid besteht, und das zweite Material ein Polymermaterial ist, das ausgewählt ist aus der Gruppe, die aus Polypropylen, Polydioxanon und Copolymeren oder Poly(glycolid-co-caprolacton) besteht.

## Revendications

1. Monofilament de prise de tissu co-extrudé (100), comprenant :
une âme (102, 302), constituée d'un premier matériau et s'étendant le long d'une longueur dudit monofilament ; et **caractérisé par**
une pluralité d'éléments de prise de tissu (106, 300) s'étendant vers l'extérieur depuis ladite âme (102, 302), au moins le long d'une partie prédéterminée de la longueur du monofilament (100), la pluralité d'éléments de prise de tissu (106, 300) étant constituée d'un deuxième matériau différent ayant une rigidité plus élevée que le premier matériau, et en ce que le premier matériau a un module initial inférieur ou égal à environ 2,76 GPa (400 kpsi) et
le deuxième matériau a un module initial d'au moins environ 3,45 GPa (500 kpsi).

2. Monofilament selon la revendication 1, le monofilament étant d'une taille adaptée pour utilisation en tant que suture chirurgicale.

3. Monofilament selon la revendication 1, le deuxième matériau entourant sensiblement l'âme (102).

4. Monofilament selon la revendication 1, la pluralité d'éléments de prise de tissu ayant chacun une partie de base (303) et une partie d'extrémité distale (304), et la partie de base (303) étant incorporée dans l'âme.

5. Monofilament selon la revendication 4, la partie de base (303) ayant une ou plusieurs saillies (306) s'étendant latéralement vers l'extérieur depuis celle-ci qui facilite le couplage mécanique des éléments de prise de tissu avec l'âme.

6. Monofilament selon la revendication 1, une section transversale de la pluralité d'éléments de prise de tissu (106, 300) diminue d'une extrémité proximale de celui-ci à une pointe distale de celui-ci la plus éloignée de ladite âme (102, 302).

7. Monofilament selon la revendication 1, la section transversale de l'âme (102, 302) étant une forme choisie dans le groupe constitué d'une forme circulaire, ovale, triangulaire et polygonale.

8. Monofilament selon la revendication 1, le premier matériau étant un matériau polymère choisi dans le groupe constitué de polyéthylène téréphtalate, et de polymères ou copolymères de lactide et glycolide.

9. Monofilament selon la revendication 8, les copolymères de lactide et glycolide étant un matériau polymère choisi dans le groupe constitué de copolymère 95/5 de poly(lactide-co-glycolide) et copolymère 90/ 10 de poly(glycolide-co-lactide).

10. Monofilament selon la revendication 8, le deuxième matériau étant un matériau polymère choisi dans le groupe constitué de polypropylène, polydioxanone, et copolymères de poly(glycolide-cocaprolactone).

11. Monofilament selon la revendication 10, le deuxième matériau étant un copolymère séquencé 75/25 de poly(glycolide-cocaprolactone).

12. Procédé pour former un monofilament comprenant les étapes de :
fourniture d'un premier matériau ayant une première rigidité dans son état solide ;
fourniture d'un deuxième matériau différent ayant une deuxième rigidité dans son état solide qui est supérieure à celle du premier matériau ;
fusion des premier et deuxième matériaux ;
co-extrusion des premier et deuxième matériaux pour former un monofilament, le premier matériau formant une âme (102, 302) du monofilament et étant **caractérisé en ce que** le deuxième matériau forme une ou plusieurs arêtes (106, 300) s'étendant vers l'extérieur au-delà d'une périphérie externe de l'âme, et
le premier matériau ayant un module initial inférieur ou égal à environ 2,76 GPa (400 kpsi), et le deuxième matériau ayant une rigidité initiale d'au moins environ 3,45 GPa (500 kpsi).

13. Procédé selon la revendication 12, le deuxième matériau du monofilament co-extrudé entourant sensiblement l'âme.

14. Procédé selon la revendication 12, une partie de base (303) de chacune de la pluralité d'arêtes (300) étant incorporée dans l'âme et une partie d'extrémité distale (304) de chacune de la pluralité d'arêtes (300) s'étendant vers l'extérieur au-delà de la périphérie externe de l'âme.

15. Procédé selon la revendication 14, la partie de base (303) de chacune de la pluralité d'arêtes (300) comprenant en outre une ou plusieurs saillies (306) s'étendant latéralement vers l'extérieur depuis celle-ci.

16. Procédé selon la revendication 12, comprenant en outre la formation d'une pluralité d'éléments de prise de tissu dans les une ou plusieurs arêtes (300, 400) en enlevant du matériau de ceux-ci à des emplacements prédéterminés.

17. Procédé selon la revendication 12, le premier matériau étant un matériau polymère choisi dans le groupe constitué de polyéthylène téréphtalate et de polymères ou copolymères de lactide et glycolide, et le deuxième matériau étant un matériau polymère choisi dans le groupe constitué de polypropylène, polydioxanone et copolymères de poly(glycolide-co-caprolactone).
